# FASCICULE DE BREVET EUROPEEN

(11) **EP 0 459 266 B1**
(45) Date de publication et mention de la délivrance du brevet: **21.12.1994**
(21) Numéro de dépôt: 91108144.6
(22) Date de dépôt: 21.05.1991
(51) Int. Cl.: A61B 17/58

(54) **Plaque vissable pour le traitement des fractures de l'apophyse odontoide de l'axis**
Schraubbare Platte für Bruchbehandlung der zahnförmigen Axisapophyse
Screwable plate for fracture treatment of the dentiform axis apophyse

(30) Priorité: 23.05.1990 FR 9006672
(43) Date de publication de la demande: 04.12.1991
(73) Titulaire: Vichard, Olivier, F-25000 Besançon (FR)
(72) Inventeur: Vichard, Olivier, F-25000 Besançon (FR)

(56) Documents cités:
- EP-A- 0 196 206
- DE-A- 3 114 872
- GB-A- 2 126 903

## Description

La présente invention est relative au traitement des fractures de l'apophyse odontoïde de l'axis, ou deuxième vertèbre cervicale C2, et notamment aux moyens de **contention** après réduction, des fragments osseux à fusionner . Elle concerne plus particulièrement une plaque de forme destinée à être fixée sur la troisième vertèbre cervicale C3 et servant de support à une vis vissée dans l'axis C2 pour y assurer la compression interfragmentaire en vue de la solidarisation des fragments osseux.

L'apophyse odontoïde ou dent de l'axis C2 est l'organe mécanique essentiel de l'articulation de l'atlas, ou première vertèbre cervicale C1 qui supporte la boîte cranienne. C'est notamment autour d'elle que se produisent tous les mouvements de rotation de la tête. Du fait de cette spécificité fonctionnelle et de sa forme correspondante, cette apophyse odontoïde est une zone de convergence des efforts accidentels affectant la colonne cervicale, donc une zone de fracture privilégiée, la fracture présentant une forme parfaitement caractéristique.

Le problème du traitement de ces fractures est loin d'être résolu de manière suffisamment fiable.

L'immobilisation des fragments osseux à solidariser par un appareillage externe conventionnel tel que minerve, par exemple, n'est utilisable qu'en cas de fracture stable, c'est-à-dire où les fragments séparés n'ont pas tendance à se déplacer après réduction, et où il n'y a ni déplacement antérieur ou postérieur de l'atlas ou C1.

Dans le cas de fractures instables, il est donc nécessaire d'avoir recours à des techniques chirurgicales. Celles-ci sont relativement nombreuses, mais toutes celles actuellement connues présentent des insuffisances ou inconvénients importants qui leur sont propres. On peut les classer en trois groupes :
Dans les arthrodèses extra-articulaires mixtes par voie cervicale postérieure, on solidarise l'atlas C1 de l'axis C2 par un cerclage métallique ou en fil non résorbable par leurs arcs vertébraux postérieurs auxquels on adjoint une greffe in situ pour qu'en cas de rupture du fil d'ostéosynthèse, la solidarisation reste assurée par la fusion biologique des deux arcs vertébraux postérieurs. Dans ces conditions, si la consolidation de l'apophyse odontoïde facturée n'intervenait pas, celle-ci serait suppléée par la pose de greffe sur les arcs postérieurs de C1 et C2. Cette association d'un cerclage et d'une greffe est très fiable mais entraine, du fait de la soudure des arcs, un déficit notable de rotation de la tête, aussi est-il difficile d'admettre cette technique invalidante. Un simple cerclage sans greffe qui n'aurait pas ce défaut reste par trop aléatoire du fait du risque de rupture du fil de cerclage.

Dans l'ostéosynthèse directe par voie transbuccale, le chirurgien, passant par la cavité buccale, incise le pharynx en arrière de la base de la langue et intervient directement sur l'apophyse odontoïde fracturée sur laquelle une plaque de consolidation est vissée. Cette technique est relativement simple et logique mais la traversée de l'oropharynx peut être à l'origine de graves complications septiques.

Dans l'ostéosynthèse par voie cervicale antérieure présterno-mastoïdienne, on accède à l'apophyse odontoïde par voie d'abord cervicale au niveau de la face antérieure du cou en passant en avant du muscle sterno-cléîdo-mastoïdien. Dans cette technique préconisée par J. BOEHLER, on procède à la compression interfragmentaire de la dent (apophyse odontoïde) par vissage opéré sous visualisation radioscopique avec amplificateur de brillance. Une ou plusieurs vis sont utilisées qui perforent ou non l'extrémité crâniale (située du côté du crâne) de la dent. Ce mode opératoire très utilisé par les praticiens, car ne présentant pas les défauts de ceux précédemment évoqués, est cependant, en son stade de développement actuel, affecté d'imperfections d'ordre mécanique, source d'un nombre non négligeable d'échecs.

La présente invention qui s'apparente à ce mode opératoire, sur lequel il va être revenu plus en détail, pour une meilleure compréhension, dans la description qui suit d'un mode préférentiel d'exécution, vise à s'affranchir des inconvénients et insuffisances qui viennent d'être évoqués.

Les caractéristiques et avantages de l'invention apparaitront à la lumière de l'exposé de l'invention qui suit et pour l'intelligence duquel on se référera aux dessins, dont :
- la figure 1 est une vue en coupe par le plan sagittal rabattu sur l'horizontale de l'axis C2 et de la troisième vertèbre cervicale C3 montrant la mise en oeuvre du dispositif selon l'invention, le côté crânial étant représenté à gauche, et le côté caudal (côté coccyx de la colonne vertébrale) vers la droite, le côté antérieur étant en haut, et le côté postérieur en bas.
- la figure 2 est une vue dans un plan frontal dans le sens antéro-postérieur des mêmes vertèbres C2, C3, auxquelles est appliqué un dispositif selon l'invention,
- la figure 3 est une coupe transversale, donc approximativement horizontale,selon le plan AA des figures 1 et 2,de la troisième vertèbre cervicale C3,
- la figure 4 est une vue en élévation dans un plan sagittal orienté comme à la figure 1 d'un dispositif selon l'invention,
- la figure 5 est une vue en coupe du même dispositif selon le plan sagittal de la figure 1,
- la figure 6 est une vue en coupe du même dispositif selon le plan BB de la figure 4,
- la figure 7 est une vue dans un plan frontal dans le sens antéro-postérieur du même dispositif,

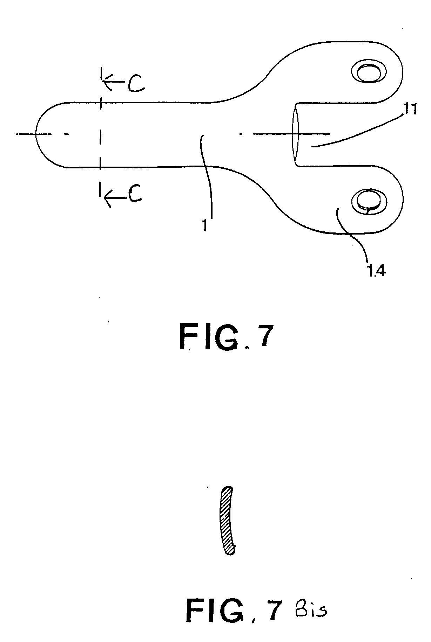

- la figure 7 bis étant une coupe selon le plan CC de la figure 7.

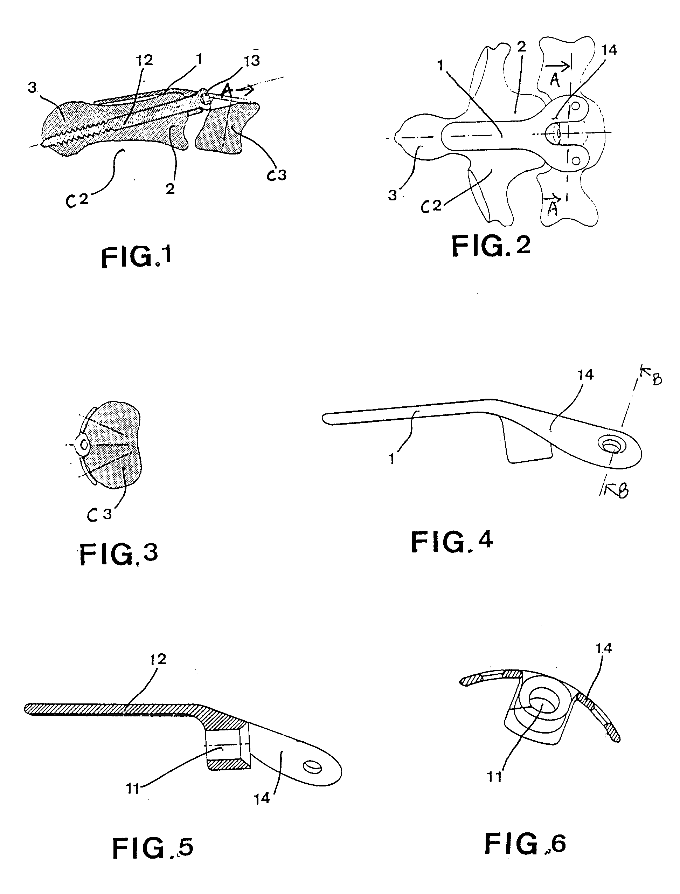

Sans qu'il y ait nécessité d'illustrations propres pour la mise en oeuvre de la technique usuelle que l'homme du métier connait et maitrise, celui-ci pourra néanmoins se reférer pour une meilleure compréhension des problèmes à résoudre, aux figures 1 et 2 explicitant la présente invention et sa mise en oeuvre.

Selon la technique usuelle, après accès, comme dit plus haut, au site de la fracture de l'apophyse odontoïde, ou dent, est normalement ménagé dans le fragment caudal de l'axis C2 ( du côté du coccyx), un avant-trou par mèche de l'ordre de 2 mm de diamètre, par exemple, dans le plan sagittal de symétrie, pour le passage d'une vis autotaraudante à extrémité lancéolée d'un diamètre d'environ 4 mm, et ce, sur une profondeur d'environ 4 à 5 mm. La vis présentant une tête, par exemple à 4 pans creux, fore elle-même son chemin dans l'avant-trou sous l'impulsion et la maitrise d'un tournevis simple et/ou à cardan, et cette progression est contrôlée par l'observation sur amplificateur de brillance. Il est alors essentiel d'obtenir une compression interfragmentaire stable, donc que la prise dans le fragment crânial de la dent, ou apophyse odontoïde, soit stable et solide et que l'extrémité de la vis franchisse le sommet crânial de cette dent.

Une seconde condition de stabilité est que la vis mise en oeuvre ne balaie pas le fragment caudal de l'axis C2 qui est de structure friable et que la tête de vis ne puisse pénétrer dans le corps de l'axis C2, ce qui supprimerait, ou du moins oblitérerait sérieusement tout effet de compression utile entre les fragments à solidariser. En général, une rondelle tend à éviter cette pénétration indésirable, mais son diamètre étant forcément limité pour des raisons d'encombrement, le résultat est loin de donner toujours satisfaction.

Ce balayage du fragment caudal de la dent de C2 est actuellement plus ou moins évité par la stabilité relative de certaines fractures qui ne présentent qu'une possibilité de déplacement dans le plan sagittal et vers l'arrière. La compression interfragmentaire empêche alors, par principe, tout déplacement dans le sens postérieur, mais ceci peut tendre à allonger la dent, et les sollicitations mécaniques qui peuvent être très importantes dans ce cas, et la rigidité du montage ne peuvent être prises en charge que par la rondelle associée à la tête de vis, et par la parfaite prise de la vis dans la partie corticale crâniale de la dent.

Dans le cas d'une instabilité sagittale antérieure ou mixte (instabilité tant dans le sens postérieur que dans le sens antérieur), la compression interfragmentaire n'a pas l'effet stabilisateur souhaité car cette compression tend à raccourcir la hauteur de la dent, conséquence intrinsèque à tout déplacement antérieur.

Il y a lieu de noter que l'instabilité éventuelle de la fracture dans le plan frontal est de moindre importance dans son amplitude par rapport aux précédentes du fait, notamment, de la grande inertie mécanique et de la disposition des masses latérales, notamment apophyses transverses, de l'atlas C1 et de l'axis C2. Cependant, cette importance n'est pas nulle du fait de l'effet de cisaillement que provoquent, même des micromouvements entre les surfaces interfragmentaires en contact, et qui est délétère pour la consolidation recherchée. Une compression interfragmentaire nettement positive pour empêcher ces microdéplacements reste le seul palliatif dans le vissage selon l'état connu de la technique actuelle, qui vient d'être exposé.

L'invention, se situant dans le cadre général du mode opératoire qui vient d'être rapidement analysé et critiqué, consiste essentiellement à supprimer la rondelle d'appui de la tête de vis pour la remplacer par une plus grande surface d'appui supprimant toute éventualité de pénétration indésirable de la tête de vis et/ou de la rondelle dans la partie caudale, friable, de l'apophyse odontoïde ou dent de l'axis C2. Dans une réalisation préférentielle, est en outre prévu un guidage positif de la vis, solidaire de la surface d'appui, pour éviter tout balayage de la vis, dans sa partie filetée ou non, dans la partie friable de l'axis C2, sans déviation radiale possible affectant le tissu osseux voisin.

Le dispositif selon l'invention, tel par exemple, qu'illustré aux dessins, se présente essentiellement sous la forme d'une plaque 1 légèrement incurvée dans les sens transversal (comme à la figure 7 bis) et longitudinal, c'est-à-dire crânial-caudal pour épouser au mieux en y prenant appui la forme du côté antérieur du fragment caudal 2 de l'axis C2 dont l'apophyse odontoïde ou dent est fracturé, le second fragment ou fragment crânial de la dent portant la référence 3.

La plaque 1 se substitue à la rondelle de l'état de la technique évoqué plus haut. Elle est munie d'un trou 11 d'axe orienté dans le sens caudal-crânial de la dent 2, 3,et de diamètre correspondant au passage d'une vis 12 en servant de surface d'appui axial à la tête 13 de cette vis. Cette plus grande surface d'appui s'oppose mieux qu'auparavant à la pénétration de la tête 13 de la vis 12 dans le fragment caudal 2 friable de l'axis C2. La vis 12,mise en place comme décrit en référence à l'état de la technique,traverse le fragment caudal 2 pour venir en prise positive dans le tissu cortical du fragment crânial 3 de C2, en franchissant de préférence le sommet de ce fragment 3. La vis 12 comporte entre sa partie terminale filetée et sa tête une partie cylindrique lisse de diamètre inférieur à celui du filetage pour ne pas interférer avec la qualité de la prise dans le fragment crânial 3.

Avantageusement, selon l'invention, la plaque d'appui 1 se prolonge du côté caudal au-delà du passage de la vis 12 par une partie galbée 14 de plan moyen incliné sur celui du reste de la plaque 1, préférentiellement en forme de fourche et venant s'assujettir sur la face antérieure de la troisième vertèbre cervicale C3 (figures 1 à 3), ce qui accroit la stabilité mécanique de l'assise de la plaque 1. L'assujettissement sur la troisième vertèbre C3 peut se faire par vissage classique, comme illustré par exemple aux figures 1 à 3.

Si la stabilité de la tête 13 de vis 12 est ainsi mieux assurée dans le sens axial, notamment contre une pénétration indésirable de la tête 13 dans le fragment caudal friable 2 de C2, la stabilité de la vis 12 continue à poser des problèmes, notamment celui d'un balayage intempestif du corps friable du fragment caudal 2 de C2. Selon l'invention, cet inconvénient est supprimé en pourvoyant la plaque d'appui 1 non plus d'un simple trou de passage, mais d'un canon de guidage 11 de la vis 12 de longueur suffisante, par exemple au moins égale au diamètre de la vis 12, empêchant toute déviation de l'axe de la vis 12 dans un plan radial quelconque. La vis 12 a alors un corps lisse de diamètre égal à celui du canon 11 et un diamètre fileté égal à celui du corps lisse, alors que jusqu'à présent, le corps lisse était de diamètre inférieur à celui du filetage, d'où les incidents de balayage déjà évoqués, du fait que la tête de vis prend maintenant appui sur la plaque 1 et non seulement une rondelle de surface très restreinte.

La plaque 1 étant par nature indéformable dans les conditions opératoires et l'angle entre les plans moyens de ses parties s'appuyant sur le fragment caudal 2 de C2 et la troisième vertèbre cervicale C3 étant fixé une fois pour toutes, tout risque de balayage dans quelque direction radiale que ce soit est donc supprimé pour la vis 12.

On voit que si l'invention supprime les inconvénients de l'état connu de la technique opératoire pour des fractures de l'apophyse odontoïde instables, elle peut aussi avantageusement s'appliquer à des fractures non déplacées et stables pour des blessés qui supporteraient très mal un appareillage externe de consolidation, ce qui est notamment le cas pour des polytraumatisés, vieillards, ou autres blessés en état général médiocre.

## Revendications

1. Dispositif pour la compression et la consolidation interfragmentaire d'une fracture de l'apophyse odontoïde (dent) de l'axis (C2) par vissage du fragment caudal (2) sur le fragment crânial (3), caractérisé par le fait qu'il est constitué d'une plaque (1) et d'une bis (12), la plaque étant conformée pour être apposée sur la face antérieure du fragment caudal (2) de la dent fracturée pour y prendre appui et comportant un trou (11) pour le passage de la vis (12) en servant d'appui axial à sa tête (13), la vis (12) étant destinée à prendre prise au moins dans le fragment crânial (3) de la dent.

2. Dispositif selon la revendication 1, caractérisé par le fait que la plaque (1) comporte un canon (11) d'axe correspondant au moins approximativement à la direction de l'axe moyen de la dent et de diamètre correspondant au diamètre extérieur de la vis (12) pour assurer la stabilité tant axiale que radiale de celle-ci dans sa progression dans les fragments caudal (2) et crânial (3) de la dent.

3. Dispositif selon la revendication 1 ou la revendication 2, caractérisé par le fait que la plaque (1) comporte une partie galbée (14) susceptible de venir s'appuyer sur, et d'être assujettie à, la surface antérieure du corps de la troisième vertèbre cervivale (C3).

4. Dispositif selon la revendication 3, caractérisé par le fait que la partie galbée (14) se présente sous la forme d'une fourche dont les branches laissent libre entre elles un espace correspondant à un accès libre au trou de passage de vis (11) ou au canon.

5. Dispositif selon la revendication 3 ou la revendication 4, caractérisé par le fait que la partie galbée (14) de la plaque (1) destinée à s'appuyer sur la troisième vertèbre cervicale (C3) est assujettie à celle-ci par vissage.

6. Dispositif selon l'une quelconque des revendications 3 à 5, caractérisé par le fait que le plan moyen de la partie galbée (14) de la plaque (1) destinée à s'appuyer sur la troisième vertèbre cervicale (C3) est incliné par rapport au plan moyen à la partie de la plaque (1) en appui sur le fragment caudal (2) de la dent selon un angle correspondant aux positions relatives normales et stables de l'axis (2) et de cette troisième vertèbre cervicale (C3).

## Claims

1. A device for the compression and interfragmentary consolidation of a fracture of the odontoid apophysis (dens) of the axis (C2) that fuses the caudal fragment with the cranial fragment, comprising:
a plate shaped to be placed on the anterior face of the caudal fragment of the fractured dens, such plate having a hole for receiving a screw, the plate serving as axial support for its head; and
a screw passing through said plate hole and secured to the cranial fragment of the dens.

2. The device according to Claim 1, wherein said plate additionally comprises a barrel having an axis corresponding approximately to the direction of the mean axis of the dens and with a diameter corresponding to the outside diameter of the screw to assure both axial and radial stability of the screw as the screw passes into the caudal fragment and cranial fragment of the dens.

3. The device according to Claim 1 wherein said plate comprises a curved portion which can be rested on and attached to the anterior surface of the body of the third cervical vertebra (C3).

4. The device according to Claim 3, wherein the curved part is forked, the branches of which leave a free space therebetween, to permit free access to the plate hole for passage of the screw or to the barrel.

5. The device according to Claim 3, comprising an additional screw, and wherein the curved part of the plate intended to rest on the third cervical vertebra (C3) is attached to the third cervical vertebra by said additional screw.

6. The device according to Claim 3 wherein the mean plane of the curved part of the plate intended to rest on the third cervical vertebra (C3) is inclined relative to the mean plane at the part of said plate resting on the caudal fragment of the dens, at an angle corresponding to the relatively normal and stable positions of the axis and of the third cervical vertebra (C3).

## Patentansprüche

1. Vorrichtung für die Kompression und die Konsolidierung der Knochenstücke bei einer Fraktur der zahnförmigen Axisapophyse (C2) durch Verschraubung des kaudalen Knochenstücks (2) an das kraniale Fragment (3). Sie wird dadurch gekennzeichnet, daß sie aus einer Platte (1) besteht, die gestaltet ist, um an der Vorderseite des kaudalen Fragments (2) der zahnförmigen Axisapophyse angesetzt zu werden und sich daran zu stützen. Diese Platte ist mit einem Loch (11) versehen zur Einführung einer Schraube (12) und als axiale Abstützung für den Schraubenkopf (13). Diese Schraube (12) soll sich wenigstens in das kraniale Fragment (3) der zahnförmigen Axisapophyse einschrauben.

2. Nach Forderung 1, Vorrichtung, die dadurch gekennzeichnet wird, daß die Platte (1) mit einem Führungslauf (11) versehen ist, dessen Achse ungefähr der Richtung der Mittellinie des Zahnes entspricht, und dessen Durchmesser dem Außendurchmesser der Schraube (12) entspricht, um ihre sowohl axiale als auch radiale Stabilität, in ihrer Progression durch das kaudale (2) und kraniale (3) Fragment des Zahns zu sichern.

3. Nach Forderung 1 oder 2, Vorrichtung, die dadurch gekennzeichnet wird, daß die Platte (1) einen geschwungenen Teil (14) enthält, der sich auf die Vorderseite des Wirbelkörpers des dritten Halswirbels (C3) stützen und an dieser befestigt werden kann.

4. Nach Forderung 3, Vorrichtung, die dadurch gekennzeichnet wird, daß der geschwungene Teil (14) unter der Form einer Gabel vorliegt, deren Zinken einen Raum freilassen, der den freien Zugang zum Durchgangsloch (11) der Schraube oder zum Führungslauf erlaubt.

5. Nach Forderung 3 oder Forderung 4, Vorrichtung, die dadurch gekennzeichnet ist, daß der geschwungene Teil (14) der Platte (1), der dazu bestimmt ist, sich auf den dritten Halswirbel (C3) zu stützen, in letzteren hineinverschraubt wird.

6. Nach irgendeiner Forderung von 3 bis 5, Vorrichtung, die dadurch gekennzeichnet wird, daß die Inklination der Mittelfläche des geschwungenen (14) Plattenteils (1), der sich auf den dritten Halswirbel (C3)stützen soll, schräg eingebogen ist in bezug auf die Mittelfläche des Plattenteils (1), der sich auf das kaudale (2) Knochenfragment der zahnförmigen Axisapophyse stützt. Der Einfallswinkel entspricht den relativen normalen, stabilen Stellungen des Axis (C2) und dieses dritten Halswirbels (C3).
